# EUROPÄISCHE PATENTSCHRIFT

(11) **EP 2 719 399 B1**
(45) Veröffentlichungstag und Bekanntmachung des Hinweises auf die Patenterteilung: **06.04.2016**
(21) Anmeldenummer: 13187960.3
(22) Anmeldetag: 09.10.2013
(51) Int. Cl.: A61L 2/08, B65B 55/08, B65B 57/02, B65B 65/00

(54) **Behältnissterilisation mittels Elektronenstrahlen mit Strahlschutz für Strahlfinger**
Container sterilisation by means of electron beams with beam protection for the beam finger
Stérilisation de récipients au moyen de faisceaux d'électrons avec protection contre le rayonnement pour les doigts de faisceaux

(30) Priorität: 09.10.2012 DE 102012109592
(43) Veröffentlichungstag der Anmeldung: 16.04.2014
(73) Patentinhaber: Krones AG, 93073 Neutraubling (DE)
(72) Erfinder: Knott, Josef, 93073 Neutraubling (DE); Scheuren, Hans, 93073 Neutraubling (DE)
(74) Vertreter: Bittner, Bernhard

(56) Entgegenhaltungen:
- EP-A1- 1 956 608
- EP-A1- 2 161 202
- EP-A2- 1 120 121
- WO-A1-2011/083106
- CH-A5- 591 991
- DE-A1-102008 007 428

## Beschreibung

Die vorliegende Erfindung bezieht sich auf eine Vorrichtung gemäß dem Oberbegriff des Anspruchs 1, und ein Verfahren zum Sterilisieren von Behältnissen gemäß dem Oberbegriff des Anspruchs 10 und wie aus der EP 1 120 121 A2 bekannt. Im Bereich der Getränke herstellenden Industrie ist es bekannt, dass diverse Getränke in sterile Behältnisse abgefüllt werden. Zu diesem Zweck sind unterschiedlichste Vorrichtungen und Verfahren zum Sterilisieren von Behältnissen bekannt. So ist eine Sterilisation mittels chemischer Substanzen, wie beispielsweise Wasserstoffperoxid oder Peressigsäure bekannt. In jüngerer Zeit ist man jedoch bestrebt, den Einsatz derartiger Chemikalien zu reduzieren.

Daher sind in jüngerer Zeit auch Sterilisationseinrichtungen bekannt geworden, welche die Behältnisse durch Beaufschlagung mit Strahlen und insbesondere mit einer Ladungsträgerstrahlung zu sterilisieren. In diesem Zusammenhang wird darauf hingewiesen, dass im Rahmen der vorliegenden Anmeldung unter Strahlung nicht nur Strahlungen im klassischen Sinne, wie beispielsweise Licht-, Röntgen-, gamma- oder UV-Strahlung verstanden werden können, sondern insbesondere auch Ladungsträgerstrahlungen, d.h. die Beaufschlagung mit Ladungsträgern, wie insbesondere aber nicht ausschließlich Elektronen. Denkbar wäre auch der Einsatz anderer Ladungsträger wie etwa alpha-Teilchen oder Protonen.

Aus dem Stand der Technik sind auch Vorrichtungen und Verfahren bekannt, bei denen in die zu sterilisierenden Behältnisse über deren Mündungen Strahlfinger eingeführt werden. Diese Strahlfinger weisen dabei üblicherweise ein Austrittsfenster für Ladungsträger, wie insbesondere Elektronen, auf. Vorteilhaft ist dabei dieses Austrittsfenster an einer Unterseite der jeweiligen Strahlfinger angeordnet. Diese Strahlfinger müssen dabei einerseits einen Querschnitt aufweisen, der durch die Mündung in das Behältnis einführbar ist. Auf der anderen Seite müssen diese Strahlfinger in ihrem Inneren üblicherweise auch ein Vakuum aufrechterhalten, innerhalb dessen die Ladungsträger beschleunigt werden können. Dies führt dazu, dass derartige Strahlfinger oftmals sehr filigrane und auch kostenintensive Geräte sind. Aufgrund der geringen mechanischen Beanspruchbarkeit sind auch die Kosten bei einer einmaligen leichten oder auch schweren Beschädigung sehr hoch.

Der vorliegenden Erfindung liegt daher die Aufgabe zugrunde, derartige Beschädigungen und insbesondere mechanische Beschädigungen der besagten Strahlerelemente zu vermeiden. Diese Aufgabe wird erfindungsgemäß durch Vorrichtungen und Verfahren nach den unabhängigen Ansprüchen gelöst. Vorteilhafte Ausführungsformen und Weiterbildungen sind Gegenstand der Unteransprüche.

Eine erfindungsgemäße Vorrichtung zum Sterilisieren von Behältnissen weist eine Sterilisationseinheit auf, welche einen stangenartigen Körper aufweist, der durch eine Mündung der Behältnisse in einen Innenraum der Behältnisse einführbar ist und der eine Innenwandung der Behältnisse mit Strahlung, insbesondere mit Ladungsträgern und insbesondere mit Elektronen beaufschlagt. Weiterhin weist die Vorrichtung eine Bewegungseinrichtung auf, welche eine Relativbewegung zwischen den Behältnissen und der Sterilisationseinheit und insbesondere dem stangenartigen Körper derart erzeugt, dass der stangenartige Körper in die Behältnisse eingeführt wird bzw. einführbar ist.

Erfindungsgemäß weist die Vorrichtung eine Kollisionsverhinderungseinrichtung auf, welche eine Kollision des stangenartigen Körpers mit dem Behältnis, insbesondere einem Bereich des Behältnisses und insbesondere der Mündung des Behältnisses, bei Auftreten einer Fehlstellung des Behältnisses und/oder einer Halteeinrichtung zum Halten des Behältnisses verhindert.

Vorteilhaft weist der stangenartige Körper insbesondere an seinem unteren d.h. dem Behältnisboden zugewandten Ende ein Austrittsfenster auf, durch welches die Ladungsträger austreten können. Bei einer bevorzugten Ausführungsform weist die Sterilisationseinheit auch eine Kühleinrichtung zum Kühlen des Austrittsfensters auf. Dabei ist es denkbar, dass der stangenartige Körper einen Kanal aufweist, durch den hindurch ein insbesondere gasförmiges Medium in Richtung des Austrittsfensters geleitet werden kann. Vorteilhaft handelt es sich bei dem Austrittsfenster um ein Austrittsfenster aus Titan, welches besonders bevorzugt eine Dicke aufweist, welche zwischen 7 und 13 µm liegt.

Bevorzugt weist die Sterilisationseinheit eine Ladungsträgererzeugungseinheit auf, sowie bevorzugt auch eine Beschleunigungseinrichtung, welche die erzeugten Ladungsträger in Richtung des Austrittsfensters beschleunigt.

Bei einer weiteren vorteilhaften Ausführungsform weist die Vorrichtung eine Transporteinrichtung auf, welche die Behältnisse während ihrer Sterilisation transportiert. Vorteilhaft weist diese Transporteinrichtung einen bewegbaren und insbesondere um eine vorgegebene Achse drehbaren Träger auf, an dem besonders bevorzugt eine Vielzahl der oben erwähnten Sterilisationseinheiten angeordnet ist.

Vorteilhaft bewegt die Bewegungseinrichtung die Behältnisse und die stangenartigen Körper sind fest an dem besagten Träger angeordnet. Damit weist bevorzugt die Vorrichtung eine Vielzahl der oben erwähnten Sterilisationseinheiten auf.

Bei einer weiteren vorteilhaften Ausführungsform weist die Vorrichtung einen Reinraum auf, bzw. bildet einen Reinraum aus, innerhalb dessen die Kunststoffbehältnisse während ihrer Sterilisation transportiert werden. Dabei ist bevorzugt dieser Reinraum mittels wenigstens einer Wandung gegenüber einer Umgebung abgedichtet.

Vorteilhaft bildet auch der besagte Träger, an dem die Sterilisationseinheiten angeordnet sind, eine Wandung dieses Reinraums aus. Bei einer weiteren vorteilhaften Ausführungsform weist die Vorrichtung eine Dichtungseinrichtung auf, um diesen Reinraum gegenüber einer Umgebung abzudichten. Bei dieser Dichtungseinrichtung kann es sich beispielsweise um ein sogenanntes Wasserschloss handeln welches bevorzugt einen mit einem flüssigen Medium befüllbaren umlaufenden Kanal aufweist, in den ein Wandungselement eintaucht und diesem gegenüber bewegbar ist.

Bei einer weiteren vorteilhaften Ausführungsform weist die Vorrichtung auch Abschirmelemente auf, um zu verhindern, dass Strahlung, insbesondere Röntgenstrahlung aus der Anlage austritt.

Bei einer weiteren vorteilhaften Ausführungsform weist die Kollisionsverhinderungseinrichtung einen Kontaktkörper auf, der- bei Auftreten einer Fehlstellung des Behältnisses und/oder einer Halteeinrichtung zum Halten des Behältnisses eine Kollision des stangenartigen Körpers mit dem Behältnis, insbesondere der Mündung des Behältnisses verhindert. Damit soll bei dieser Ausführungsform insbesondere eine Kollision des stangenartigen Körpers mit dem Behältnis selbst und/oder dessen Halteeinrichtung verhindert werden.

In der Praxis kann es dazu kommen, dass die Halte- oder Greifelemente, welche die Behältnisse halten, diese nicht ordnungsgemäß zu fassen bekommen und die Behältnisse beispielsweise schief in den Halteelementen liegen. In dieser Situation ist es denkbar, dass bei der Zustellbewegung des Behältnisses zu dem stangenartigen Körper dieser stangenartige Körper mit dem Behältnis kollidiert. Bereits diese Kollision kann ausreichen, um den stangenartigen Körper nachhaltig zu beschädigen.

Durch den besagten Kontaktkörper kann eine derartige Kollision verhindert werden. So ist es möglich, dass die Vorrichtung bei Auftreten eines derartigen mechanischen Kontaktes zwischen dem Behältnis und dem Kontaktkörper die Zustellbewegung anhält. Es wäre jedoch auch möglich, dass in Reaktion auf einen derartigen Kontakt das Halteelement das Behältnis loslässt, sodass dieses nicht mehr mit dem stangenartigen Körper in Kontakt treten kann.

Bevorzugt ist daher dieser Kontaktkörper derart angeordnet, dass der Kontaktkörper das Behältnis kontaktiert, bevor der stangenartige Körper das Behältnis kontaktiert. Vorteilhaft handelt es sich bei dem Behältnis um ein Kunststoffbehältnis und besonders bevorzugt um einen Kunststoffvorformling. Die Erfindung wäre jedoch auch auf Kunststoffflaschen anwendbar.

Bei einer weiteren vorteilhaften Ausführungsform weist der Kontaktkörper einen rohrförmigen Körper auf, der den stangenartigen Körper wenigstens abschnittsweise umgibt. Durch diesen rohrförmigen Körper wird der stangenartige Körper einerseits in dessen radialer Richtung gegen Stöße geschützt, andererseits ist es möglich, dass dieser rohrförmige Körper insbesondere schief sitzende Kunststoffbehältnisse berührt, bevor der stangenartige Körper diese berührt.

Vorteilhaft weist dieser rohrförmige Körper eine Ausnehmung bzw. einen Schlitz auf. Während der Zustellbewegung der Kunststoffbehältnisse kann ein Element der Halteeinrichtung, welche die Kunststoffbehältnisse hält, in diesen Schlitz eintreten. Bevorzugt erstreckt sich dieser Schlitz in einer Längsrichtung des stangenartigen Körpers, bei der es sich bevorzugt auch um eine Bewegungsrichtung des Behältnisses gegenüber dem stangenartigen Körper handelt. Es wäre dabei möglich, dass der Kontaktkörper als Edelstahlrohr oder allgemein Stahlrohr ausgeführt ist, welches eine vorgegebene Materialdicke aufweist. Vorteilhaft ist der Kontaktkörper aus einem vergleichsweise soliden Material hergestellt, sodass es auch Kollisionen mit der Halteeinrichtung und/oder dem Behältnis standhält.

Bei einer weiteren vorteilhaften Ausführungsform ist eine Schalteinrichtung vorgesehen, welche bewirkt, dass bei einem Kontakt zwischen dem Kontaktkörper und dem Kunststoffbehältnis und/oder der Halteeinrichtung für das Kunststoffbehältnis die Zustellbewegung des Kunststoffbehältnisses angehalten wird. Vorteilhaft ist eine Ausschleuseeinrichtung vorgesehen, welche fehlerhaft an der Halteeinrichtung angeordnete Behältnisse ausschleust. Insbesondere ist diese Ausschleuseeinrichtung in der Transportrichtung der Kunststoffbehältnisse nach einem Abschnitt des Transportpfades in der Sterilisationsanordnung angeordnet, in welchem Abschnitt die Sterilisation im regulären Betrieb stattfindet und vor einer Übergabeposition, an der das Behältnis an eine weiterführende Transporteinrichtung übergeben wird. Dadurch wird vorteilhaft vermieden, dass ein gegebenenfalls fehlerhaft ausgerichtetes Behältnis bei Verlassen der Sterilisationsanordnung auch wieder fehlerhaft ausgerichtet an eine weiterführende Transporteinrichtung wie etwa ein Abführrad übergeben wird, was üblicherweise zu Kollisionen und Maschinenstillstand führt.

Bei einer weiteren vorteilhaften Ausführungsform ist ein Innenquerschnitt des rohrförmigen Körpers größer als ein Außenquerschnitt des Behältnisses. Vorteilhaft ist der Innenquerschnitt des rohrförmigen Körpers geringfügig größer als der Außenquerschnitt des Behältnisses, sodass dieses im Wesentlichen nur in einer vertikalen Anordnung bzw. korrekten Ausrichtung in diesen rohrförmigen Körper eingeschoben werden kann. Falls es sich bei den zu behandelnden Behältnissen um Kunststoffvorformlinge handelt, ist damit bevorzugt dieser rohrförmige Körper in seinem Querschnitt etwas größer als der Tragring der Kunststoffvorformlinge. Je weniger dieser Tragring über den eigentlichen Kunststoffvorformling hervorsteht, desto kleiner wird bevorzugt auch der Durchmesser dieses rohrförmigen Körpers ausgeführt und desto besser ist auch die Schutzfunktion, da bereits bei geringen Fehlstellungen des Kunststoffvorformlings ein Anschlagen an den rohrförmigen Körper erreicht wird. Daneben ist es auch möglich, die Tiefe des Rohrs oder damit auch den Schutzbereich geringer einzustellen, wenn dieser Schutzkörper bzw. der rohrförmige Körper (in radialer Richtung) nahe an dem stangenartigen Körper angeordnet ist.

Bevorzugt ist der rohrförmige Körper in der Längsrichtung des stangenartigen Körpers länger als der stangenartige Körper und/oder reicht näher an das Kunststoffbehältnis heran als der stangenartige Körper. Bevorzugt ist daher der stangenartige Körper vollständig in einem durch den rohrförmigen Körper im Wesentlichen in Umfangsrichtung umschlossenen Hohlraum angeordnet. Bevorzugt ist der stangenartige Körper nur aus der Richtung, in der auch das Behältnis angeordnet ist, zugänglich (beispielsweise nur von unten her).

Vorteilhaft weist der rohrförmige Körper einen kreisförmigen Querschnitt auf. Bevorzugt ist der stangenartige Körper symmetrisch innerhalb des rohrförmigen Körpers angeordnet. Bei einer weiteren bevorzugten Ausführungsform sind der rohrförmige Körper und der stangenartige Körper fest bezüglich einander angeordnet.

Bei einer weiteren vorteilhaften Ausführungsform weist die Vorrichtung überdies auch ein Strahlungsminderungselement auf, welches die auf einen vorgegebenen Bereich des Behältnisses und insbesondere auf einen Mündungsbereich der Behältnisse auftretenden Strahlungsanteile vermindert. Ein Problem bei der Verwendung ionisierender Strahlung ist die mögliche Überdosierung des Halsbereiches. Dies ist dadurch gegeben, dass der Halsbereich im Vergleich zu dem Rest des Kunststoffvorformlings beim An- und Wegfahren des Kunststoffvorformlings an den bzw. von dem Emitter eine sehr hohe Strahlungsdosis abbekommt.

Es wird darauf hingewiesen, dass dieses Strahlungsminderungselement auch unabhängig von dem oben erwähnten Schutzmechanismus ausführbar ist. Die Anmelderin behält sich daher vor, Schutz zu beanspruchen für eine Vorrichtung zum Sterilisieren von Behältnissen mit einer Sterilisationseinheit, welche einen stangenartigen Körper aufweist, der durch eine Mündung der Behältnisse in einen Innenraum der Behältnisse einführbar ist und welche eine Innenwandung der Behältnisse mit einer Strahlung beaufschlagt sowie einer Bewegungseinrichtung, welche eine Relativbewegung zwischen den Behältnissen und der Sterilisationseinheit derart erzeugt, dass der stangenartige Körper in die Behältnisse eingeführt wird. Bei dieser Ausführungsform wird erfindungsgemäß eine Strahlungsminderungseinrichtung bzw. ein Strahlungsminderungselement vorgesehen, welches den auf einen vorgegebenen Bereich des Behältnisses auftretenden Strahlungsanteil vermindert.

Vorteilhaft ist das oben erwähnte Strahlungsminderungselement ringförmig ausgebildet und bevorzugt wenigstens zeitweise in einem Bereich der Mündung des Behältnisses angeordnet. Auf diese Weise kann dieses Strahlungsminderungselement einen Teil derjenigen Strahlen abfangen, welche ansonsten auf den Mündungsbereich des Behältnisses gelangen würde. Dabei ist vorteilhaft dieses Strahlungsminderungselement als Metallring ausgeführt. Es wäre dabei auch möglich, dass dieses Strahlungsminderungselement geladen ist, um so Ladungsträger anzuziehen. Vorteilhaft ist dieses Strahlungsminderungselement auch in den oben erwähnten rohrförmigen Körper einführbar.

Vorteilhaft weist das Strahlungsminderungselement einen Querschnitt auf, der größer ist als ein Mündungsquerschnitt des zu sterilisierenden Behältnisses. Vorteilhaft ist das Strahlüngsminderungselement während des Sterilisationsvorgangs geringfügig oberhalb eines Mündungsrandes des zu sterilisierenden Behältnisses angeordnet. Vorteilhaft beträgt während der Sterilisation des Behältnisses ein Abstand in der Längsrichtung zwischen 0,1cm und 5cm, bevorzugt zwischen 0,2cm und 5cm, bevorzugt zwischen 0,3cm und 4cm und bevorzugt zwischen 1cm und 4cm.

Vorteilhaft ist eine Bewegung dieses Strahlungsminderungselements mit einer Bewegung einer Halteeinrichtung zum Halten der Behältnisse gekoppelt. So könnte beispielsweise eine Greifeinrichtung vorgesehen sein, welche die Behältnisse greift und welche mit der besagten Strahlungsminderungseinrichtung an einen gemeinsamen Träger angeordnet ist.

Bei einer weiteren vorteilhaften Ausführungsform weist die Kollisionsverhinderungseinrichtung eine Bildaufnahmeeinrichtung aufweist, welche eine Relativposition des Behältnisses erfasst. So kann beispielsweise mittels einer Kamera erfasst werden, ob die Behältnisse korrekt an ihren Halteeinrichtungen angeordnet sind. Dazu können die mit der Bildaufnahmeeinrichtung erfassten Bilder beispielsweise mit einem Referenzbild verglichen werden. Diese optische Erfassung der korrekten Anordnung der Behältnisse an ihren Halteeinrichtung kann anstelle der oben erwähnten Vorgehensweise oder auch zusätzlich zu dieser angewandt werden.

So kann der oben beschriebene mechanische Kollisionsschutz auch um eine optische Lösung erweitert werden. Hierfür wird vorgeschlagen, eine Kamera -vorteilhaft im Übergabebereich zwischen Zuführeinheit und Karussell zur Innenbehandlung - zu positionieren. Diese ist bevorzugt mit einer Auswerteeinheit verbunden und kontrolliert die Position zwischen Klammer und Preform, indem eine Aufnahme gemacht wird, die mit einem vorgegebenen Standard vergleichen wird.

Tritt hier eine Abweichung auf, so wird bevorzugt die Preform zwar übergeben, die anschließende Führung der Preform oder des Behälters im allgemeinen wird jedoch verzögert, so dass die zu erwartende Kollision mit dem Schutz in seiner Heftigkeit vermindert wird. Dies bedeutet einen besseren Schutz des empfindlichen Strahlers und eine reduzierte mechanische Belastung des Strahlerschutzes. Es wäre jedoch auch möglich, ein als fehlerhaft orientiert erkanntes Behältnis auszuwerfen.

Vorteilhaft ist die Bildaufnahmeeinrichtung stationär angeordnet, etwa seitlich an einem Transportpfad der Behältnisse.

Die vorliegende Erfindung ist weiterhin auf ein Verfahren zum Sterilisieren von Behältnissen gerichtet, wobei die Behältnisse entlang eines vorgegebenen Transportpfades transportiert werden und während dieses Transportes sterilisiert werden, und wobei zum Sterilisieren der Behältnisse ein stangenartiger Körper in die Behältnisse eingeführt wird und im Inneren der Behältnisse eine Innenwandung der Behältnisse mit Ladungsträgern beaufschlagt. Dabei werden die Behältnisse in einer Längsrichtung der Behältnisse bezüglich des stangenartigen Körpers bewegt, um den stangenartigen Körper in das Behältnis einzuführen.

Erfindungsgemäß wird mittels einer Kollisionsverhinderungseinrichtung eine Kollision zwischen dem stangenartigen Körper und dem Behältnis verhindert.

Es wird daher auch verfahrensseitig vorgeschlagen, dass eine Kollision zwischen dem stangenartigen Körper, im Folgenden auch als Strahlfinger bezeichnet, und dem Behältnis verhindert wird. Insbesondere wird dabei eine Kollision verhindert, wenn das Behältnis in einer Fehlstellung angeordnet ist.

Bei einem weiteren vorteilhaften Verfahren wird die Kollision durch einen mechanischen Kontakt zwischen dem Behältnis und einem Bestandteil der Kollisionsverhinderungseinrichtung verhindert.

Bei einem weiteren vorteilhaften Verfahren wird wenigstens ein Abschnitt des Behältnisses und insbesondere zumindest ein Mündungsabschnitt des Behältnisses in die Kollisionsverhinderungseinrichtung bzw. einen Bestandteil der Kollisionsverhinderungseinrichtung eingeführt.

Dabei kann diese Kollisionsverhinderungseinrichtung, wie oben erwähnt, einen rohrförmigen Körper aufweisen, in den das Behältnis einführbar ist.

Vorteilhaft werden mittels dieses erfindungsgemäßen Verfahrens Kunststoffvorformlinge sterilisiert und insbesondere eine Innenwandung der Kunststoffvorformlinge sterilisiert. Vorteilhaft wird mittels einer Strahlungsminderungseinrichtung eine auf einen vorgegebenen Bereich des Behältnisses und insbesondere einen Mündungsbereich des Behältnisses aufgebrachte Strahlungsdosis vermindert. Dabei kann es sich, wie oben erwähnt, um einen Ring handeln, der in einem Bereich der Mündung angeordnet ist und so einen Teil der auf das Behältnis auftreffenden Strahlung abfängt.

Bei einer weiteren vorteilhaften Ausführungsform wäre es auch möglich, dass berührungslos und insbesondere optisch und bevorzugt mittels einer Bildaufnahmeeinrichtung eine Positionierung der Behältnisse an deren Trägern erfasst wird. So wäre es möglich, dass festgestellt wird, ob die Behältnisse korrekt an ihren Halteeinrichtungen angeordnet sind.

Weitere Vorteile und Ausführungsformen ergeben sich aus den beigefügten Zeichnungen:

### Darin zeigen:

- Fig. 1: eine schematische Darstellung einer Anlage zum Sterilisieren von Behältnissen;
- Fig. 2: eine Detailansicht einer Vorrichtung zum Sterilisieren von Behältnissen nach dem Stand der Technik;
- Fix. 3: eine erste schematische Darstellung einer erfindungsgemäßen Vorrichtung;
- Fig. 4: die Vorrichtung aus Fig. 3 in einem weiteren Zustand;
- Fig. 5: die Vorrichtung aus Fig. 3 in einem weiteren Zustand;
- Fig. 6: die erfindungsgemäße Vorrichtung bei Fehlstellung eines Kunststoffvorformlings; und
- Fig. 7: die Vorrichtung aus Fig. 6 in einem weiteren Zustand.

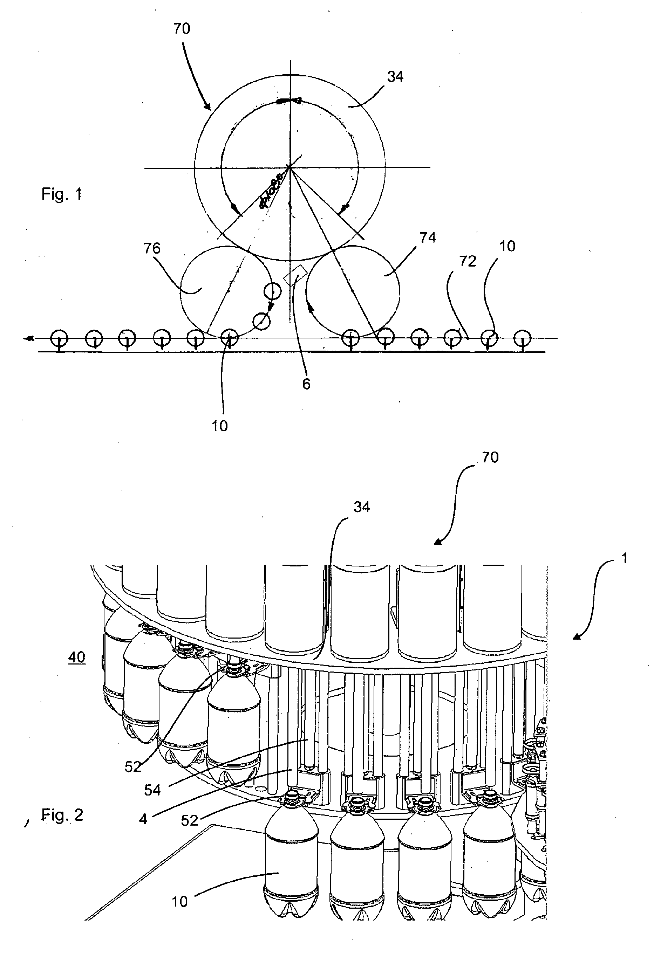

Figur 1 zeigt eine Darstellung einer Vorrichtung zum Sterilisieren von Behältnissen.

Dabei ist eine Zuführeinrichtung 72 gezeigt, welche Kunststoffbehältnisse 10, hier Kunststoffvorformlinge, über ein Zuführrad 74 einer Sterilisationsanordnung 70 zuführt. Diese Sterilisationsanordnung weist einen drehbaren Träger 34 auf, an der eine Vielzahl von (nicht gezeigten) Sterilisationsvorrichtungen angeordnet ist. Dabei weisen diese Sterilisationsvorrichtungen hier stangenartige Körper auf, die in das Innere der Kunststoffbehältnisse 10 einführbar sind.

Über ein Abführrad 76 werden die auf diese Weise sterilisierten Kunststoffbehältnisse 10 wieder aus der Vorrichtung abgeführt.

Das Bezugszeichen 6 bezieht sich auf eine Bildaufnahmeeinrichtung, die optional in einem Bereich zwischen dem Zuführrad und der Sterilisationseinrichtung 70 angeordnet ist. Diese kann optisch Fehlstellungen der Behältnisse an deren Halteeinrichtungen erfassen.

Figur 2 zeigt eine Detaildarstellung der Sterilisationsanordnung 70 nach dem internen Stand der Technik der Anmelderin. Man erkennt, dass in dieser Sterilisationsanordnung eine Vielzahl von Sterilisationsvorrichtungen 1 angeordnet ist. Diese Sterilisationsvorrichtungen 1 weisen dabei jeweils stangenartige Körper bzw. Strahlfinger 4 auf, die in das Innere der Behältnisse 10 einführbar sind. In Figur 2 sind als Behältnisse Kunststoffflaschen dargestellt, wie oben erwähnt könnte es sich hier jedoch bei den Behältnissen auch (bevorzugt) um Kunststoffvorformlinge handeln. Die stangenartigen Körper 4 (bzw. Strahlfinger), an deren unterem Ende sich jeweils das (nicht gezeigte) Austrittsfenster befindet, werden in das Innere des Behältnisses 10 eingeführt. Zu diesem Zweck wird das Behältnis 10 über eine Halteeinrichtung 52, wie etwa einen Greifer, gehalten und über eine Bewegungseinrichtung 54 (beispielsweise einen Linearmotor) angehoben.

Bei einer bevorzugten Ausführungsform ist die Sterilisationsanordnung 70 derart ausgeführt, dass die Kunststoffbehältnisse innerhalb eines Reinraumes 40 transportiert werden. Dabei kann der Träger 34 selbst bereits eine Wandung dieses Reinraums 40 ausbilden. Weiterhin sind bevorzugt Dichtungseinrichtungen vorgesehen, welche den Träger 34 bzw. eine bewegliche Wandung dieses Reinraumes 40 gegenüber einer unbeweglichen Wandung abdichten. Bei der in Figur 2 gezeigten Ausführungsform sind vorteilhaft Bestandteile der jeweiligen Sterilisationseinrichtungen, wie insbesondere die Elektronenerzeuger und die Elektronenbeschleuniger, außerhalb des in seiner Gesamtheit mit 40 bezeichneten Reinraums angeordnet.

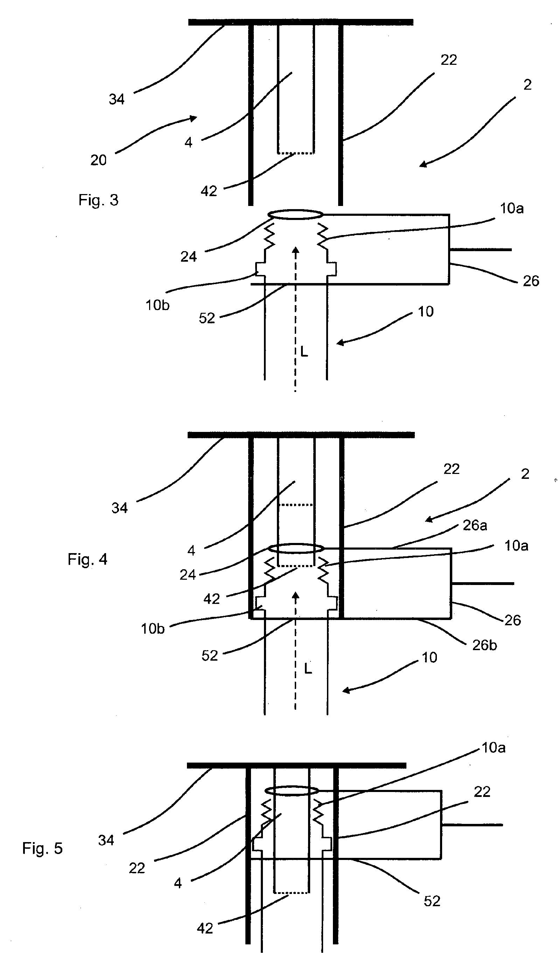

Figur 3 zeigt eine schematische Darstellung einer erfindungsgemäßen Sterilisationseinheit 2. Diese Sterilisationseinheit weist einen stangenartigen Körper 4 auf, an dessen unterem Ende ein Austrittsfenster 42 angeordnet ist, über welches die Elektronen austreten können. Das Bezugszeichen 34 kennzeichnet wieder den Träger, an dem eine Vielzahl stangenartiger Körper 4 angeordnet ist. Dabei sind die stangenartigen Körper 4 jeweils fest an dem Träger 34 angeordnet, d.h. sie bewegen sich nicht in der Längsrichtung L der Kunststoffvorformlinge 10.

Das Bezugszeichen 20 bezieht sich auf eine Kollisionsverhinderungseinrichtung in ihrer Gesamtheit. Diese Kollisionsverhinderungseinrichtung 20 weist hier einen an dem Träger 34 angeordneten rohrförmigen Körper 22 auf, der den stangenartigen Körper 4 in dessen Umfangsrichtung im Wesentlichen umgibt. Dabei schützt dieser rohrförmige Körper 22 den stangenartigen Körper einerseits vor Impulsen, die in einer radialen Richtung auf den stangenartigen Körper auftreffen können. Daneben wirkt, wie im Folgenden gezeigt wird, dieser rohrförmige Körper 22 auch als Kollisionsverhinderungseinrichtung, wenn die Kunststoffvorformlinge gegenüber ihrer Sollorientierung schief angeordnet sind.

Das Bezugszeichen 26 kennzeichnet hier einen Träger, an dem ein Greifelement 52 (nur schematisch dargestellt) angeordnet ist, welches den Kunststoffvorformling unterhalb seines Tragringes 10b und damit auch unterhalb seiner Mündung 10a halten kann. An diesem Träger 26 ist weiterhin eine Strahlungsverminderungseinrichtung bzw. Abschirmeinrichtung 24 angeordnet, die damit fest in Bezug auf das Greifelement 52 angeordnet ist. Diese Strahlungsverminderungseinrichtung 24 ist derart angeordnet, dass sie unmittelbar oberhalb der Mündung des Behältnisses liegt. Auf diese Weise kann bei einem Einführen des stangenartigen Körpers in das Kunststoffbehältnis bzw. wieder aus diesem heraus Elektronenstrahlung abgefangen werden, so dass diese nicht in einem Übermaß an die Mündung des Kunststoffvorformlings gelangt.

Figur 4 zeigt eine weitere Position der erfindungsgemäßen Vorrichtung. Hier ist der Kunststoffvorformling 10 mit seinem Greifer 52 nach oben gefahren, so dass die Mündung 10a nunmehr bereits in den rohrförmigen Körper 22 eintaucht. Man erkennt in allen Figuren, dass der rohrförmige Körper über den stangenartigen Körper 4 nach unten heraussteht, um diesen zu schützen. Bei der in Figur 4 gezeigten Darstellung ist der Kunststoffvorformling regulär angeordnet und wird damit in den rohrförmigen Körper 22 eingeführt, ohne diesen zu berühren. Gleichzeitig taucht hier bereits auch der stangenartige Körper 4 in die Mündung 10a des Kunststoffvorformlings ein. Die Strahlungsverminderungseinrichtung 24 ist, wie oben erwähnt, als ringförmiges Element ausgebildet, durch welches der stangenartige Körper 4 hindurchtritt.

Bei der in Fig. 5 gezeigten Situation ist das Kunststoffbehältnis noch weiter in den rohrförmigen Körper 22 eingefahren In dem rohrförmigen Körper 22 ist ein in der Längsrichtung L verlaufender Schlitz (nicht sichtbar) angeordnet, durch den ein Bestandteil des Trägers 26 wie etwa die Halteabschnitte 26a, 26b geführt werden können. Hier ist weiterhin auch der stangenartige Körper noch weiter in den Kunststoffvorformling 10 eingefahren. Das vollständige Greifelement 52 ist hier ebenfalls innerhalb des rohrförmigen Körpers 22 angeordnet.

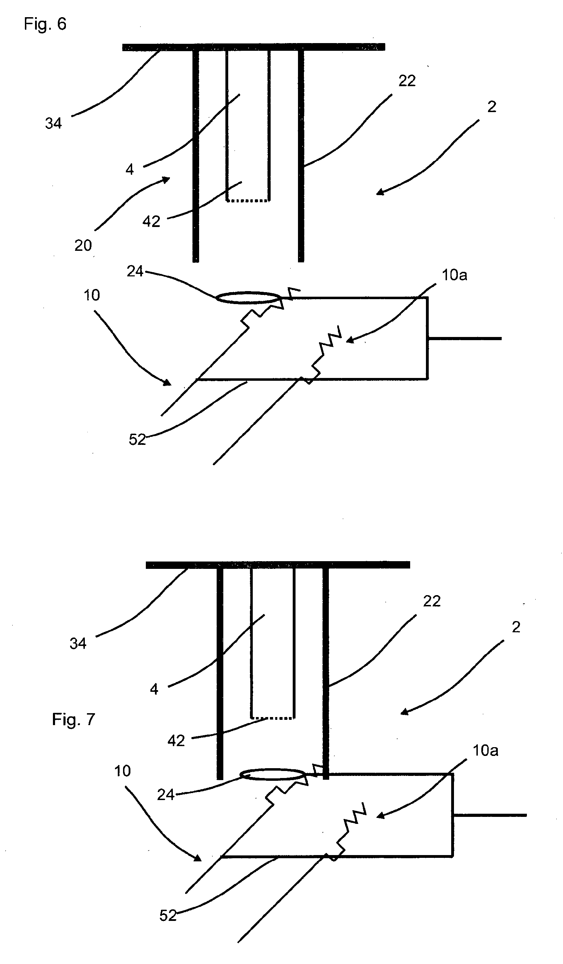

Figur 6 zeigt eine Darstellung, bei der der Kunststoffvorformling schief in seinem Greifelement 52 sitzt. Bei der in Figur 6 gezeigten Situation ist es noch nicht zu einer Kollision gekommen. In dieser Situation wird das Greifelement 52 noch in Richtung des stangenartigen Körpers 4 angehoben.

Bei der in Figur 7 gezeigten Situation kontaktiert ein Bereich der Mündung 10a des Kunststoffvorformlings 10 einen Bereich des rohrförmigen Körpers 22. In dieser Situation kann beispielsweise die weitere Bewegung des Greifelementes 52 in der Längsrichtung L angehalten werden, so dass eine Kollision zwischen dem Kunststoffvorformling und dem stangenartigen Körper 4 verhindert wird. Gleichzeitig bewirkt der stabilere rohrförmige Körper 22, dass in jedem Falle ein weiteres Anheben des Kunststoffvorformlings in der Längsrichtung L nicht möglich ist, so dass auf diese Weise wirksam die Kollision zwischen dem Behältnis 10 und dem stangenartigen Körper 4 verhindert werden kann.

Alternativ zu dem beschriebenen rohrförmigen Körper wäre ebenso denkbar, dass die Kollisionsverhinderungseinrichtung aus beispielsweise zwei konzentrisch zur Rotationsachse des drehbaren Trägers angeordneten im Wesentlichen zylindrischen Wandungen (nicht gezeigt) gebildet wird. Eine der Wandungen kann radial außerhalb des Teilkreises der stangenartigen Körper liegen, eine weitere kann radial innerhalb dieses Teilkreises liegen. Auch könnten die stangenartigen Körper derart an dem drehbaren Träger angeordnet sein, dass deren unteres, das Austrittsfenster aufweisende Fenster nicht aus dem Träger heraussteht. Das heißt, die stangenartigen Körper wären in diesem Fall im Träger eingebettet und der drehbare Träger selbst dient als Kollisionsverhinderungsvorrichtung.

Anstelle des in den obigen Figuren gezeigten rohrförmigen Körpers 22 könnte auch eine Vielzahl von stangenartigen Körpern, die beispielsweise entlang einer Kreislinie angeordnet sind, vorgesehen sein. Zwischen diesen Stangen könnten (in Umfangsrichtung bzw. entlang dieser Kreislinie Abstände ausgebildet sein, bzw. die stangenartigen Körper könnten kammartig ausgebildet sein. Im Schadensfall können einzelne verbogene Stangen ausgetauscht werden Anstatt eine komplette Schutzeinheit zu wechseln, wären somit nur einzelne kleinere Schutzeinheiten zu bewegen.

Hier wäre weiterhin als vorteilhaft zu berücksichtigen, dass auch die Zugänglichkeit am Strahler dadurch verbessert wäre. Etwaige Wartungs- oder Reinigungstätigkeiten wären einfacher durchzuführen. Bevorzugt sind damit diese einzelnen stangennartigen Körper einzeln von einem Träger entfernbar. Diese stangeartigen Körper könnten dabei bajonettverschlussartig auf einem (insbesondere gemeinsamen) Träger aufgesteckt oder auch eingedreht sein. Auch könnten diese stangenartigen Körper teleskopartig ausgebildet sein.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind.

### Bezugszeichenliste

- 2: Sterilisationseinheit
- 4: stangenartiger Körper
- 10: Behältnis
- 10a: Mündung des Behältnisses
- 10b: Tragring
- 20: Kollisionsverhinderungseinrichtung
- 22: rohrförmiger Körper
- 24: Strahlungsverminderungseinrichtung
- 26: Träger
- 26a, 26b: Halteabschnitte
- 34: Träger
- 40: Reinraum
- 42: Austrittsfenster
- 52: Halteeinrichtung
- 54: Bewegungseinrichtung
- 70: Sterilisationsahordnung
- 72: Zuführeinrichtung
- 74: Zuführrad
- 76: Abführrad

- L: Längsrichtung

## Patentansprüche

1. Vorrichtung (1) zum Sterilisieren von Behältnissen (10), mit einer Sterilisationseinheit (2), welche einen stangenartigen Körper (4) aufweist, der durch eine, Mündung der Behältnisse (10) in einen Innenraum der Behältnisse (10) einführbar ist und welcher eine Innenwandung der Behältnisse (10) mit einer Strahlung beaufschlagt und mit einer Bewegungseinrichtung (54), welche eine Relativbewegung zwischen den Behältnissen (10) und der Sterilisationseinheit (2) derart erzeugt, dass der stangenartige Körper (4) in die Behältnisse (10) einführbar ist,
**dadurch gekennzeichnet, dass**
die Vorrichtung (1) eine Kollisionsverhinderungseinrichtung (20) aufweist, welche eine Kollision des stangenartigen Körpers (4) mit dem Behältnis (10) bei Auftreten einer Fehlstellung des Behältnisses (10) und/oder einer Halteeinrichtung (52) zum Halten des Behältnisses (10) verhindert.

2. Vorrichtung (1) nach Anspruch 1,
**dadurch gekennzeichnet, dass**
die Kollisionsverhinderungseinrichtung (20) einen Kontaktkörper (22) aufweist, der bei Auftreten einer Fehlstellung des Behältnisses und/oder einer Halteeinrichtung (52) zum Halten des Behältnisses (10) eine Kollision des stangenartigen Körpers (4) mit dem Behältnis und/oder der Halteeinrichtung (52) zum Halten des Behältnisses (10) verhindert.

3. Vorrichtung (1) nach Anspruch 1,
**dadurch gekennzeichnet, dass** der Kontaktkörper (22) das Behältnis (10) berührt, bevor der stangenartige Körper (4) das Behältnis (10) kontaktiert.

4. Vorrichtung (1) nach Anspruch 2,
**dadurch gekennzeichnet, dass**
der Kontaktkörper (22) einen rohrförmigen Körper (22) aufweist, der den stangenartigen Körper (4) wenigstens abschnittsweise umgibt.

5. Vorrichtung (1) nach Anspruch 2,
**dadurch gekennzeichnet, dass**
ein Innenquerschnitt des Kontaktkörpers (22) größer ist als ein Außenquerschnitt des Behältnisses (10).

6. Vorrichtung (1) nach wenigstens einem der vorangegangenen Ansprüche,
**dadurch gekennzeichnet, dass**
die Vorrichtung (1) ein Strahlungsminderungselement (24) aufweist, welches den auf den Mündungsbereich der Behältnisse (10) auftretenden Strahlungsanteil vermindert.

7. Vorrichtung (1) nach Anspruch 6,
**dadurch gekennzeichnet, dass**
das Strahlungsminderungselement (24) ringförmig ausgebildet ist und bevorzugt wenigstens zeitweise in einem Bereich der Mündung des Behältnisses (10) angeordnet ist.

8. Vorrichtung (1) Anspruch 6,
**dadurch gekennzeichnet, dass**
eine Bewegung des Strahlungsminderungselements (24) mit einer Bewegung einer Halteeinrichtung (52) zum Halten der Behältnisse (10) gekoppelt ist.

9. Vorrichtung (1) nach Anspruch 1,
**dadurch gekennzeichnet, dass**
die Kollisionsverhinderungseinrichtung (20) eine Bildaufnahmeeinrichtung (6) aufweist, welche eine Relativposition der Behältnisse erfasst.

10. Verfahren zum Sterilisieren von Behältnissen (10), wobei die Behältnisse (10) entlang eines vorgegebenen Transportpfades transportiert werden und während dieses Transports sterilisiert werden, wobei zum Sterilisieren der Behältnisse (10) ein stangenartiger Körper (4) in die Behältnisse (10) eingeführt wird und im Inneren der Behältnisse (10) eine Innenwandung der Behältnisse (10) mit Ladungsträgern beaufschlagt und wobei die Behältnisse in einer Längsrichtung (L) der Behältnisse (10) bezüglich des stangenartigen Körpers (4) bewegt werden, um den stangenartigen Körper (4) in das Behältnis (10) einzuführen,
**dadurch gekennzeichnet, dass**
mittels einer Kollisionsverhinderungseinrichtung (20) eine Kollision zwischen dem stangenartigen Körper (4) und dem Behältnis (10) bei Auftreten einer Fehlstellung des Behältnisses (10) und/oder einer Halteeinrichtung (52) zum Halten des Behältnisses (10) verhindert wird.

11. Verfahren nach Anspruch 10,
**dadurch gekennzeichnet, dass**
wenigstens ein Abschnitt des Behältnisses (10) in die Kollisionsverhinderungseinrichtung (20) eingeführt wird.

12. Verfahren nach Anspruch 10,
**dadurch gekennzeichnet, dass**
mittels einer Strahlungsminderungseinrichtung (24) eine auf einen Mündungsbereich (10a) des Behältnisses (10) aufgebrachte Strahlungsdosis vermindert wird.

## Claims

1. An apparatus (1) for the sterilization of containers (10), with a sterilization unit (2) which has a rod-like body (4) which rod-like body is capable of being introduced through an aperture of the containers (10) into an interior space of the containers (10) and which acts upon an inner wall of the containers (10) with radiation, and with a movement device (54) which produces a relative movement between the containers (10) and the sterilization unit (2) in such a way that the rod-like body (4) is capable of being introduced into the containers (10), **characterized in that** the apparatus (1) has a collision-prevention device (20) which prevents a collision of the rod-like body (4) with the container (10) when an incorrect positioning of the container (10) and/or a holding device (52) for holding the container (10) occurs.

2. An apparatus (1) according to claim 1, **characterized in that** the collision-prevention device (20) has a contact body (22) which - when an incorrect positioning of the container and/or a holding device (52) for holding the container (10) occurs - prevents a collision of the rod-like body (4) with the container and/or the holding device (52) for holding the container (10).

3. An apparatus (1) according to claim 1, **characterized in that** the contact body (22) contacts the container (10) before the rod-like body (4) contacts the container (10).

4. An apparatus (1) according to claim 2, **characterized in that** the contact body (22) has a tubular body (22) which surrounds the rod-like body (4) at least in sections.

5. An apparatus (1) according to claim 2, **characterized in that** an internal cross-section of the contact body (22) is larger than an external cross-section of the container (10).

6. An apparatus (1) according to at least one of the preceding claims, **characterized in that** the apparatus (1) has a radiation reduction element (24) which reduces the amount of radiation occurring on an aperture region of the containers (10).

7. An apparatus (1) according to claim 6, **characterized in that** the radiation reduction element (24) is made annular and is preferably arranged at least for a time in a region of the aperture of the container (10).

8. An apparatus (1) according to claim 6, **characterized in that** a movement of the radiation reduction element (24) is coupled to a movement of a holding device (52) for holding the containers (10).

9. An apparatus (1) according to claim 1, **characterized in that** the collision-prevention device (20) has an image-recording device (6) which records a relative position of the containers.

10. A method of sterilizing containers (10), wherein the containers (10) are conveyed along a pre-set conveying path and are sterilized during this conveying, wherein, in order to sterilize the containers (10), a rod-like body (4) is introduced into the containers (10) and an inner wall of the containers (10) is acted upon with charge carriers in the interior of the containers (10) and wherein the containers are moved in a longitudinal direction (L) of the containers (10) with respect to the rod-like body (4) in order to introduce the rod-like body (4) into the container (10), **characterized in that** a collision between the rod-like body (4) and the container (10) is prevented by means of a collision-prevention device (20) when an incorrect positioning of the container (10) and/or a holding device (52) for holding the container (10) occurs.

11. A method according to claim 10, **characterized in that** at least one portion of the container (10) is introduced into the collision-prevention device (20).

12. A method according to claim 10, **characterized in that** a radiation dose applied to an aperture region (10a) of the container (10) is reduced by means of a radiation reduction device (24).

## Revendications

1. Dispositif (1) pour la stérilisation de récipients (10), avec une unité de stérilisation (2) présentant un corps en forme de barre (4), lequel peut être introduit à l'intérieur des récipients (10) par une embouchure des récipients (10) et applique un rayonnement sur une paroi intérieure des récipients (10), et avec un moyen de déplacement (54), lequel génère un déplacement relatif entre les récipients (10) et l'unité de stérilisation (2), de telle manière que le corps en forme de barre (4) peut être introduit dans les récipients (10),
**caractérisé en ce que**
ledit dispositif (1) comporte un moyen de prévention de collision (20) empêchant une collision du corps en forme de barre (4) avec le récipient (10) en cas de position erronée du récipient (10) et/ou d'un moyen de maintien (52) destiné à maintenir le récipient (10).

2. Dispositif (1) selon la revendication 1,
**caractérisé en ce que**
ledit moyen de prévention de collision (20) comporte un corps de contact (22) empêchant une collision du corps en forme de barre (4) avec le récipient et/ou le moyen de maintien (52) destiné à maintenir le récipient (10) en cas de position erronée du récipient et/ou du moyen de maintien (52) destiné à maintenir le récipient (10).

3. Dispositif (1) selon la revendication 1,
**caractérisé en ce que**
ledit corps de contact (22) touche le récipient (10) avant que le corps en forme de barre (4) contacte le récipient (10).

4. Dispositif (1) selon la revendication 2,
**caractérisé en ce que**
ledit corps de contact (22) comporte un corps tubulaire (22), qui entoure au moins en sections le corps en forme de barre (4).

5. Dispositif (1) selon la revendication 2,
**caractérisé en ce qu'**
une section transversale intérieure du corps de contact (22) est supérieure à une section transversale extérieure du récipient (10).

6. Dispositif (1) selon au moins une des revendications précédentes, **caractérisé en ce que**
ledit dispositif (1) comporte un élément réducteur de rayonnement (24) qui limite la part de rayonnement incident sur la zone d'embouchure des récipients (10).

7. Dispositif (1) selon la revendication 6,
**caractérisé en ce que**
ledit élément réducteur de rayonnement (24) est prévu de forme annulaire et est de préférence disposé au moins temporairement dans une zone de l'embouchure du récipient (10).

8. Dispositif (1) selon la revendication 6,
**caractérisé en ce qu'**
un déplacement de l'élément réducteur de rayonnement (24) est couplé à un déplacement du moyen de maintien (52) destiné à maintenir les récipients (10).

9. Dispositif (1) selon la revendication 1,
**caractérisé en ce que**
ledit moyen de prévention de collision (20) comporte un moyen de capture d'image (6) qui détecte une position relative des récipients.

10. Procédé de stérilisation de récipients (10), où les récipients (10) sont convoyés le long d'un chemin de transport défini et stérilisés pendant ce transport, un corps en forme de barre (4) étant introduit dans les récipients (10) pour la stérilisation des récipients (10) et sollicitant une paroi intérieure des récipients (10) avec des porteurs de charge à l'intérieur des récipients (10), et où les récipients sont déplacés suivant une direction longitudinale (L) des récipients (10) par rapport au corps en forme de barre (4), pour introduire le corps en forme de barre (4) dans le récipient (10),
**caractérisé en ce qu'**
une collision entre le corps en forme de barre (4) et le récipient (10) est empêchée par le moyen de prévention de collision (20) en cas de position erronée du récipient (10) et/ou du moyen de maintien (52) destiné à maintenir le récipient (10).

11. Procédé selon la revendication 10,
**caractérisé en ce qu'**
au moins une section du récipient (10) est introduite dans le moyen de prévention de collision (20).

12. Procédé selon la revendication 10,
**caractérisé en ce qu'**
une dose de rayonnement appliquée sur une zone d'embouchure (10a) du récipient (10) est limitée au moyen d'un moyen réducteur de rayonnement (24).
